# EUROPEAN PATENT SPECIFICATION

(11) **EP 2 464 379 B1**
(45) Date of publication and mention of the grant of the patent: **30.07.2014**
(21) Application number: 10807829.6
(22) Date of filing: 10.08.2010
(51) Int. Cl.: A61K 39/39, A61K 39/145, A61P 31/12

(54) **Vaccine having a peptide adjuvant for eliciting a specific immune response to treat influenza viral infection**
Impfstoff mit einem Peptidadjuvans zur Herbeiführung einer spezifischen Immunreaktion zur Behandlung von Influenzavirusinfektionen
Vaccin ayant un adjuvant peptidique pour induire une réponse immunitaire spécifique afin de traiter une infection virale de l'influenza

(30) Priority: 10.08.2009 US 232618 P
(43) Date of publication of application: 20.06.2012
(73) Proprietor: Immunotech Developments Inc., Mississauga, Ontario L5K 1B3 (CA)
(72) Inventor: SAHNER, David, Santa Cruz California 95060 (US); LALONDE, Guy, Woodside California 94062 (US)
(74) Representative: Hart, Deborah Mary
(86) International application number: PCT/CA2010/001225
(87) International publication number: WO 2011/017799

(56) References cited:
- EP-A1- 1 840 133
- WO-A1-2008/014613
- WO-A2-94/20063
- CA-A1- 2 276 542
- US-A- 6 159 940
- US-A- 6 159 940

## Description

### TECHNICAL FIELD

This invention is related to the field of vaccine development: specifically a peptide adjuvant in a vaccine to promote and enhance the immune response in a subject who has been administered the vaccine for prophylactic or therapeutic treatment of infection or disease.

### BACKGROUND

Vaccines are used to elicit a specific immune response against a particular target antigen. For example, vaccines against viral or bacterial components are used to prevent or limit infection caused by the respective pathogen. Vaccines against tumor specific antigens or a combination of such antigens are used in the treatment of cancer. However, to an unprimed immune system, target antigens are typically poor at stimulating a specific immune response on their own, especially in vaccines where the immunizing antigen is an isolated or synthesized peptide. To overcome this, commercial vaccine preparations typically contain not just the target antigen, but also an immunological adjuvant.

The adjuvant may promote an improved immune response in one or more of several ways: for example, promoting antigen delivery to or activation of antigen presenting cells, stimulating lymphocytes, inducing a local influx of inflammatory cells, or providing a durable reservoir of antigen. Specific adjuvants may promote polarization of Th1 (cellular) or Th2 (humoral) responses, and increase the magnitude or durability of the immune response.

Adjuvants made from aluminum salts (aluminum hydroxide or aluminum phosphate) have been in widespread use for decades in prophylactic vaccines for various infectious diseases. They promote a Th2 regulated immune response, where the humoral (antibody) component predominates over the cellular component. With the advent of highly purified protein and subunit vaccines, as well as DNA-based vaccines, there is renewed interest in developing effective and well-tolerated vaccine adjuvants. For established vaccines, improved adjuvants may allow the use of a smaller quantity of immunogen per dose - potentially extending immunization coverage to wider segments of the global population.

New adjuvants are being sought for vaccines designed for cancer treatment, because cancer results in an impairment of dendritic cell maturation and function. This compromises antigen presentation, and may also be associated with activation of immunosuppressive regulatory T cells. Melacine® (a vaccine targeting tumor antigens CHER-2/neu and L523S in melanoma) contains the adjuvant ASO4, which is a combination of the monophosphoryl lipid A derivative MPL and an aluminum salt. ASO4 is also used as adjuvant in Fendrix™ (Boland et al., Vaccine 2004; 23:316-320), which has been approved as a Hepatitis B vaccine in Europe.

Inactivated influenza vaccine reduces the incidence of laboratory-confirmed influenza in 70 to 90% of adults under 65 years of age - but among persons over 65, vaccine efficacy estimates range from 43-56% when the antigenic match between circulating and vaccine strains is optimal, and only 21-42% when strains diverge antigenically. This is a considerable problem, because the morbidity and mortality of influenza is especially severe amongst the elderly.

Recently, regulatory approval has been given overseas for the influenza vaccine Fluad®, which is formulated with the adjuvant MF-59™, an oil-in-water emulsion composed of squalene and two types of surfactant. Compared with the standard influenza vaccine, Fluad may elicit a stronger humoral (antibody) response. Older patients who receive Fluad® are significantly less likely to require hospitalization during peak virus circulation (Joan Puig-Barberà et al., Vaccine 25 (2007) 7313-7321). However, although MF-59 is generally very well-tolerated, it has also been linked to malaise and a substantial increase in local vaccine reactions compared with conventional vaccine (Minutello et al., Vaccine. 1999 Jan;17(2):99-104).

### Previous vaccine compositions containing peptides

Chedid et al. (Infect Immun. 1982 Feb;35(2):417-24) described biological activity of a synthetic muramyl peptide adjuvant. U.S. Patent 4,094,971 provides a water-soluble product that is supposed to have immunological activity in vivo when administered to a host in an oil-free aqueous solution. The product is an acylated peptidoglycan fragment having saccharide units of N-acylmuramyl and N-acetylglucosamine. U.S. Patent 4,094,971 provides a water-soluble product that is supposed to have immunological activity in-vivo when administered to a host in an oil-free aqueous solution.

More recently, Schmidt et al. did experiments to develop a cancer vaccine by transloading tumor cells with foreign major histocompatibility complex class I peptide ligand (Proc Natl Acad Sci U S A. 1996 Sep 3;93(18):9759-63). Reidl et al. (Eur J Immunol. 2002 Jun;32(6):1709-16) have said that binding immune-stimulating oligonucleotides to cationic peptides from viral core antigen enhances their potency as adjuvants. U.S. Patent application US 2009/0123486 A1 outlines a vaccine having an antigen and a peptide enriched in positively charged natural and/or non-natural amino acid residues, particularly a combination of lysine and leucine.

Duryee et al. (Vaccine. 2009 May 14;27(22):2981-8) generated immune responses to methamphetamine by active immunization with vaccines containing an adjuvant based on a 9-amino acid peptide. Kobiyama et al. (J Immunol. 2009 Feb 1;182(3):1593-601) showed that a signaling polypeptide derived from an innate immune adaptor molecule can be harnessed as a new class of vaccine adjuvant. U.S. Patent application US 2008/0311138 A1 provides an immunogenic composition containing a particular gastrointestinal peptide adjuvant.

Lingnau et al (Expert Rev Vaccines 2007 Oct,6(5) 741-6) have reviewed the subject of vaccine adjuvant based on toll-like receptor agonists. Takeshita et al (J Virol 2006 Jul,80(13) 6218-24) did experiments to show that toll-like receptor adaptor molecules enhance DNA-raised adaptive immune responses against influenza and tumors through activation of innate immunity.

### Previous clinical uses of synthetic peptides

In unrelated work, small oligopeptides have been developed for use in other types of clinical therapy.

US Patent 6,184,208 describes peptides having the formula X-Tyr-Y-Phe-Z-A In this formula, X is Arg, D-Arg, D-ornithine, homoarginine, D-homoarginine, or citrulline, Y is D-ornithine, D-Ala, or D-Arg, Z is D-Ala, Gly, Pro, D-Pro or b-alanine, and A is -OH or -NH₂. Exemplary is a peptide having the sequence H-Arg-Tyr-(D-Ala)-Phe-Gly-OH (Fleishman et al , Bull Exp Biol Med 2007 Sep, 144(3) 309-11) These peptides are being developed under the trade name Dermorphin™ for stimulating hair growth, weight gain, wound healing, and reparative and anabolic processes Dermorphin analogs incorporating a stabilizer ring have been tested for analgesic, opioid, and adjuvant activities (WO 2008/014613).

US Patent 6,410,515 describes peptides having the formula X-A-(D-Trp)-Y, where X, A, and Y are each chosen from a particular list of alternative amino acids or other groups. Exemplary is a peptide having the sequence H-(D-isoglutamic acid-(D-Trp)-OH (Semina et al , Bull Exp Biol Med 2008 Jul, 146(1) 96-9) These peptides are being developed as immunosupressants under the trade name Thymodepressin™.

US Patents 6,051 ,683 and 6,159,950 along with Canadian patent application 2,276,542 describe a separate family of peptides having the formula X-Glu-Trp-Y. These peptides have the ability to promote colony formation in a CFU-S assay, and were developed for use in hematopoiesis in the context of cancer therapy Exemplary is a peptide having the sequence H-Ile-Glu-Trp-OH (Dambaeva et al, Zh Mikrobiol Epidemiol Immunobiol 2002 Nov-Dec,(6) 55-9 and Ziablitskii et al, Radiats Biol Radioecol 2003 Jan-Feb,43(1) 49-50), which has been developed under the trade name Neogen™. Another series of compounds is described in WO 2009/065217 in which Glu is joined to Trp by way of the Glu gamma carboxyl group These peptides have been developed to treat a deficiency in hematopoiesis by oral administration under the trade name IsoNeogen™.

WO 94/20063 discloses the use of Glu-Trp (IM862) together with an anti-flu vaccine. The use of IM862 decreased the sickness rate, and in the event of flu the course of infection was noted to be less severe and the recovery rate more rapid as compared to controls.

### SUMMARY OF THE INVENTION

This invention addresses the need for new adjuvants that intensify or modulate the character of the immune responses generated by vaccine compositions. The invention is suitable both for protection against infections agents, and the treatment of existing disease caused by infectious agents and cancer. The vaccines of this invention are suitable for use in a wide range of human patients and have special advantages for treatment of the elderly and patients who are immunocompromised.

One embodiment of this invention is an immunogenic composition or vaccine. The components are an influenza antigen against which the response is desired, and an oligopeptide having the formula Ile-Glu-Trp. The oligopeptide acts as an adjuvant to promote a specific immune response against the antigen in the composition. The antigen and oligopeptide are typically dissolved or suspended in a convenient amount of liquid for administration, prepared under sterile and purity conditions according to regulatory review for human treatment.

The antigen is an influenza antigen. It may be present as isolated peptides, or as part of a live, attenuated, or inactivated microbial particle or extract. Exemplary is an inactivated influenza vaccine containing one or more epitopes from neuraminidase or hemagglutinin of several strains of Influenza A, and optionally Influenza B or Influenza C.

In another embodiment of this invention the immunogenic composition can be used in a method of eliciting a specific immune response against an antigen in a subject by administering an immunogenic composition of this invention. The composition may be more effective than previous vaccines where the subject is elderly or immunocompromised, or where a rapid T-cell response is desired. One way to boost the immune response is to administer an antigen-oligopeptide combination, and then administer the oligopeptide without the antigen on at least two successive occasions within about 5 days afterwards. In order to make this type of therapy available to the treating physician, the compositions of the invention may be distributed in kit form: for example, a vaccine composition containing the target antigen and the oligopeptide adjuvant in one container, and the oligopeptide alone in another container.

In another embodiment of this invention the immunogenic composition is used in the preparation of a medicament for eliciting a specific immune response against the antigen. In another embodiment of this invention the immunogenic composition is for use in a method for treating a disease or infection in which said antigen is a component, or for generating a specific Th1 or cellular response against the antigen.

Other embodiments of the invention will be apparent from the description that follows.

### DRAWINGS

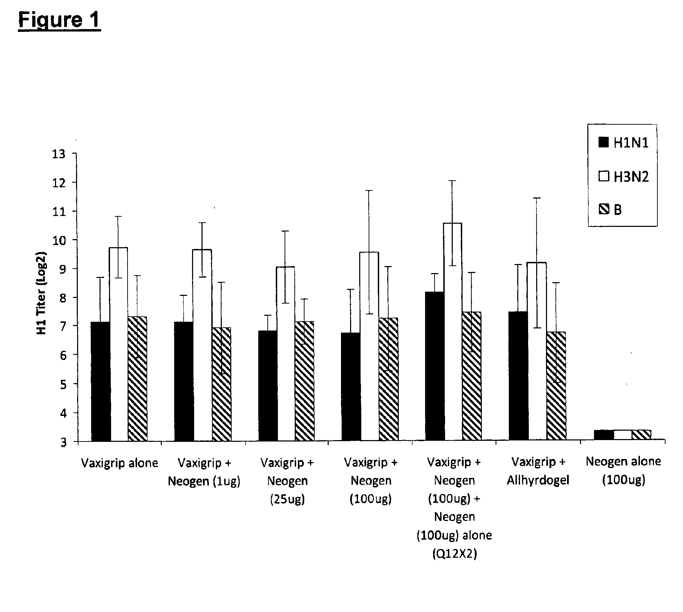

FIG. 1 shows the results from a mouse model experiment in which an immunostimulatory tripeptide was tested for its ability to augment a specific immune response against human influenza. Titers were determined in a hemagglutination inhibition (HI) assay, a measure of induced antibodies to the influenza hemagglutinin surface antigen (mean ± standard deviation). There was no HI titer in mice receiving Neogen alone, showing that the peptide does not stimulate the immune response in a non-specific manner. Mice that received Vaxigrip plus Neogen, and then 2 follow-up injections of Neogen alone, had a higher HI response.

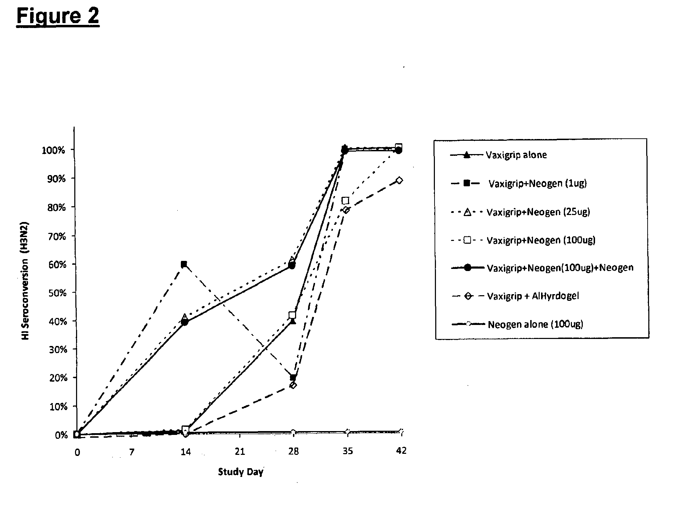

FIG. 2 shows the kinetics of H3N2 seroconversion, as each animal attained an HI titer that was four-fold increase from baseline. Three of the Neogen adjuvant groups showed earlier seroconversion of a larger proportion of animals than either the flu antigen (Vaxigrip) alone, or the Alhydrogel® (aluminum hydroxide) composition.

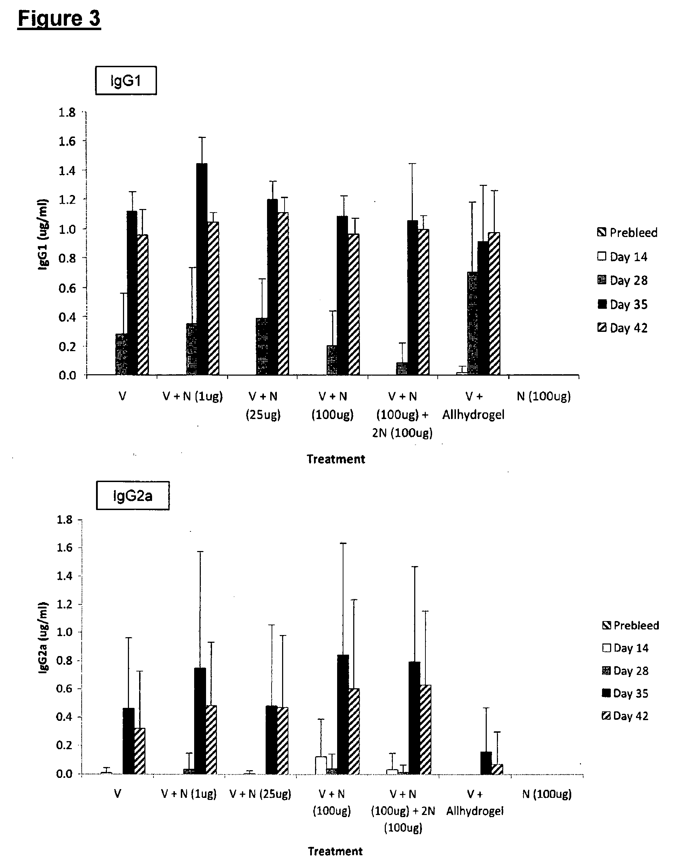

FIG. 3 shows the IgG1 and IgG2a antibody response to influenza antigen, as determined by ELISA (Upper and Lower Panels, respectively). Specific IgG1 is generally associated with a Th2 regulated response, whereas specific IgG2a is generally associated with a Th1 regulated response, which is generally accompanied by cellular immunity. With 100 µg of Neogen in the composition, the Th1 response was substantially higher.

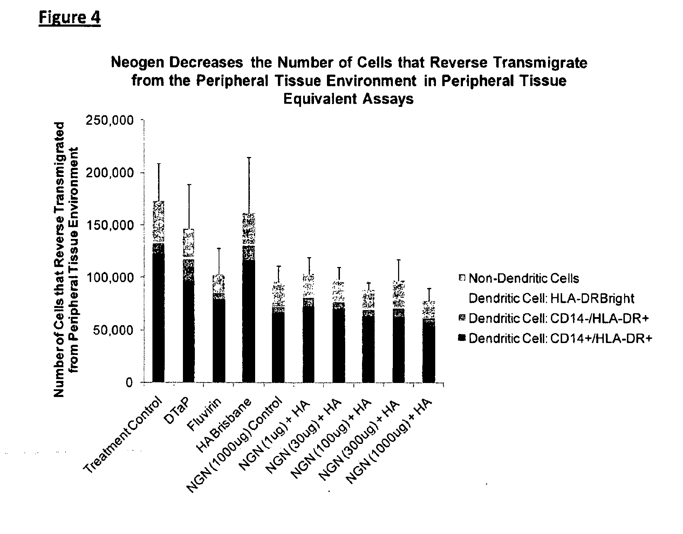

FIG. 4 shows the number of cells that reverse transmigrate from the pheripheral tissue environment in peripheral tissue equivalent assays. Neogen alone, or Neogen in the presence of antigen reduced the number of cells found to reverse transmigrate across a layer of human endothelial cells, suggesting more of the peripheral blood mononuclear cells remained in the peripheral tissue environment, prolonging dendritic cell maturation time and or differentiation into other cell types, such as macrophages. Importantly, the dendritic cells recovered from samples exposed to both Neogen and antigen were primed for antigen presentation, as indicated by expression of the cell surface antigen presenting protein HLA-DR, and an increase in the HLA-DR^{Bright}. Taken together, these data suggest that Neogen's utility as an adjuvant can be attributed to its ability to prime the innate immune system, in addition to its ability to enhance antibody production.

### DETAILED DESCRIPTION

This disclosure describes for the first time how a family of peptides previously developed for promoting hemopoiesis can be used instead as an adjuvant in vaccine compositions.

It has now been discovered that administering a target antigen in conjunction with the Neogen specific immunological response against that antigen. This places in the hands of the reader the ability to make an immunogenic or vaccine composition by combining a target antigen with a peptide in the

Neogen family The peptide can be used as an alternative to or in conjunction with other types of adjuvants such as aluminum salts, oil emulsions, and those referred to in the Background section above. Neogen helps stimulate a rapid and specific immune response with a low side-effect profile. In some contexts, the amount of Neogen in the composition can be adjusted to promote a stronger Th1 response than is obtained using conventional vaccines.

In its role as adjuvant, Neogen has a special ability to promote a specific immunological response in subjects that might otherwise be relatively unresponsive to a particular target antigen. Thus, Neogen would be an advantage over other adjuvants where the antigen used to evoke the response is poorly immunogenic. This can occur, for example, where the antigen is a small peptide or combination of peptides, or where it closely resembles an autoantigen (for example, in the case of a case of a tumor-associated antigen). It can also occur when the subject being treated is relatively unresponsive: for example, because of a concurrent infection, because of an immunodeficiency, because of increased immune tolerance, because of age, or because of a concurrent treatment that is immunocompromising (for example, for cancer).

It has also been discovered that administration of the compositions of this invention can be further optimized to improve the response against a relatively non-immunogenic antigen, or in a relatively immunocompromised subject by including Neogen not just in the vaccine composition with the antigen, but in follow-up injections of Neogen alone, for example, at or near the same injection site shortly following the vaccine. This is believed to help recruit and/or stimulate antigen presenting cells and responding leukocytes in a way that boosts the resulting specific immune response.

The peptides described in US Patents 6,051 ,683 and 6, 159,950 and in WO 2009/065217 have previously been used to promote hemopoiesis in a subject needing blood reconstitution, such as patients undergoing radioablation or other types of chemotherapy. The peptide stimulates production of various hematopoietic cells- both erythrocytes and leukocytes- in the treated subject. Thus, animals5 first irradiated and then treated with Neogen had more rapidly restored hemoglobin levels (U S Patent 6,159,950, Example 8). They had more hematopoietic progenitors, as shown by an increase in spleen colony forming units (CFU-S) (Examples 5 and 7). Irradiated mice treated with Neogen also responded to a subsequent challenge with sheep erythrocytes by making antibody forming cells (AFC) against the challenge (Example 4). This shows that the peptide stimulates broad spectrum reconstitution of hematopoietic cell function in a non-specific manner.

However, the ability of Neogen to specifically stimulate an immune response against a specific antigen target coadministered with the peptide was not previously known.

### The adjuvant peptide

As a general class, peptide adjuvants suitable for use in this invention have the formula Ile-Glu-Trp.This formula refers to peptides made from L-amino acids (except where D-amino acids are explicitly evoked) from the N- to C- terminals. These peptides and their manufacture are described in U S Patents 6,051 ,683 and 6, 159,950.

Generally, the peptide bond between Glu and Trp in the general formula can be from either the alpha or gamma carboxyl group on the Glu residue to the alpha amino group on Trp. It has been determined that joining Glu to Trp by way of the gamma carboxyl group is useful where the peptide is administered orally (WO 2009/065217). For use in vaccines of this invention administered by injection, the prototype adjuvant peptide is Ile-Glu-Trp, where the oligopeptide has a peptide bond between the alpha carboxyl group on Glu and the amino group on Trp.

The term "Neogen" as used in this description refers to the exemplary peptide Ile-Glu-Trp. For convenience, the term is used to illustrate various ways of preparing and using the vaccine compositions of the invention. Any embodiments of the invention described and illustrated in this disclosure may be practiced with any of the adjuvant peptides referred to in this section and their equivalents that have the desired properties, except were expressly limited to peptides having a particular sequence. Particular peptides falling within the generic formula and their equivalents can be tested for use in this invention by implementing the assessment procedures described below.

### The target antigen

The antigen included in the vaccine compositions of this invention will be an influenza antigen.

For example, the antigen may be an infectious agent, either live, attenuated, or inactivated, or a homogenate or protein extract thereof. Alternatively, it may be a particular protein component, an epitope of a protein, or a mixture or combination of peptides or epitopes associated with the agent.

Particularly one or more strains of human influenza A, B, C, or combinations thereof may be present. Suitable preparations include attenuated or extracted viruses, or immunogenic components of the virus, especially the surface proteins hemagglutinin and neuraminidase. These proteins undergo antigenic drift caused by cumulative mutations, and recombine with homologous viruses to undergo antigenic shift. Change in the antigenicity may render the virus transparent or less susceptible to the immune system of someone who is immune to a previous strain. For this reason, the influenza vaccine is updated regularly, and it is recommended that it be readministered on a yearly basis, particularly to elderly, people at risk for complications because of underlying medical conditions, people who are immunocompromised, and people with a high exposure rate such as health care workers. The biology and genetics of the influenza virus is described in Influenza Virology Current Topics by Y, Kawaoka, Caister Academic Press 2006. Use of flu antigens in immunogenic compositions is generally described in Vaccines for Pandemic Influenza, R. W. Compans & R W Orenstein eds , Springer 2009, and Influenza Vaccines for the Future, R Rappuoli & G Del Giudice eds , Birkhauser Base 2008.

### Types of vaccine

Because the adjuvant peptides of this invention may act by recruiting and activating antigen presenting and immune cells, in principle, they can be used to enhance the immunogenicity of a variety of different types of vaccine preparations This includes live or attenuated infectious agents, extracts, isolated proteins and mixtures thereof, peptide epitopes and mixtures thereof, naked nucleic acid vaccines and vector-delivered nucleic acid-based vaccines, cellular vaccines, and dendritic cell vaccines.

Peptide antigens can be prepared by solid-phase chemical synthesis. The principles of solid phase chemical synthesis can be found in Bioorganic Chemistry, Dugas & Penney eds , Springer- Verlag N Y pp 54-92, 1981 , and U S Patent No 4,493,795. Longer polypeptides are conveniently obtained by expression cloning. A polynucleotide encoding the desired polypeptide is operatively linked to control elements for transcription and translation, and then transfected into a suitable host cell. Expression may be effected in prokaryotes such as E coli (ATCC Accession No 31446 or 27325), eukaryotic microorganisms such as Pichia pastoris yeast, or higher eukaryotes, such as insect or mammalian cells. A number of expression systems are described in U S Patent No 5,552,524 Expression cloning is available from such commercial services as Lark Technologies, Houston TX, and AthenaES, Baltimore MD. The protein is purified from the producing host cell by standard methods in protein chemistry, such as affinity chromatography and HPLC.

Alternatively, the antigen can be produced *in situ* by administering a polynucleotide encoding it. The antigen encoding sequence is operatively linked to control elements for transcription and translation in human cells. It is then provided in a form that will promote entry and expression of the encoding sequence in cells at the disease site. Forms suitable for local injection include naked DNA, polynucleotides packaged with cationic lipids, and polynucleotides in the form of viral vectors (such as adenovirus, adeno-associated virus, and herpes virus constructs). Further information on the preparation and use of polynucleotides for therapeutic purposes is described in DNA-Pharmaceuticals: Formulation and Delivery in Gene Therapy, DNA Vaccination and Immunotherapy, M. Schleef ed., Wiley-VCH 2005.

In another alternative, the antigen can be pre-loaded into antigen presenting cells, particularly dendritic cells: either derived from the patient's own leukocytes, or as a stock medicament prepared from one or more universal donors. The cells are prepared by culturing in a combination of cytokines such as GM-CSF and IL-4, and then loaded with the antigen in peptide form, or as DNA or mRNA encoding it. See U.S. Patent Nos. 6,440,735; 7,060,279; and 7,198,948, and the textbooks Dendritic Cells in Clinics, M. Onji, Springer 2008; Macrophages and Dendritic Cells: Methods and Protocols, N.E. Reiner ed., Humana Press 2009.

In the current working embodiments of the invention, the adjuvant is provided as a chemically synthesized peptide. Any means of providing or delivering the peptide to the site of administration in combination with the antigen target can be used. Non-limiting examples of peptide delivery means include peptides in a slow-release form, and peptides generated in situ, for example, by protein cleavage or enzymatic synthesis.

The vaccine is assembled by combining the antigen source (the peptide, protein, polynucleotide, antigen presenting cells, or combination thereof) with the adjuvant peptide or peptide providing means in a suitable medium or vehicle. The ingredients are compounded into a medicament in accordance with generally accepted procedures for the preparation of pharmaceutical preparations, as described in standard textbooks on the subject. See, for example, Pharmaceutical Preformulation and Formulation: A Practical Guide from Candidate Drug Selection to Commercial Dosage Form, M. Gibson ed., Informa Health Care 2009; Pharmaceutical Manufacturing Handbook: Production and Processes, S.C. Gad ed., Wiley-interscience 2008; and the latest edition of Remington's Pharmaceutical Sciences, Maack Publishing Co, Easton PA.

Steps in the compounding or formulating of the medicament depend in part on the intended use and mode of administration, and may include sterilizing, mixing with appropriate non-toxic and non-interfering excipients, buffers and other carriers, lyophilizing or freezing, dividing into dose units, and enclosing in a delivery device. The medicament will typically be packaged in a suitable container accompanied by or associated with written information about its intended use, such as the infectious condition or other disease to be prevented or treated, and aspects of dosing and administration.

### Use of the vaccine composition

The manner in which the immunogenic compositions of this invention are used will depend on the nature of the vaccine and the disease that is the focus of the treatment.

Generally, the vaccine will be administered intramuscularly, subcutaneously, intravenously, intranasally, or orally, as appropriate, at a dosage and on a schedule determined empirically to provide the desired response in a suitable cross-section of the treated patient population.

For purposes of prophylaxis against an infectious agent, if the subject is adequately primed (such as with an annual influenza vaccination), a single administration of the antigen-adjuvant combination may be sufficient. Multiple administrations are more typical in an immunologically naive host or for a less immunogenic antigen. Desirable outcomes include induction or enhancement of a specific antibody response measured by a suitable test, such as enzyme-linked immunosorbant assay (ELISA), or (in the case of influenza) by hemagglutination inhibition (HI) assay.

For purposes of treatment or eradication of an ongoing infections disease, multiple administrations of the antigen-adjuvant composition (at least 2 or 4, for example, on a biweekly schedule) may be helpful. Here, the objective may be not just to elicit specific antibody, but also to elicit a specific T-lymphocyte response (measured in an ELISPOT or proliferation assay), or a cytotoxic T cell response (measurable, for example, in a cytotoxicity assay). Clinical benefit would be manifest as a reduction in the titer of virus or infectious particles in blood or in a tissue biopsy, or a limitation in the progression of necrosis, pain, wasting, or other signs of the disease.

To boost the immune response in any of these contexts, administration of the antigen-adjuvant composition may be preceded by or following administration of Neogen: for example on one, two, or more than two occasions within two to five days before and/or following administration of the antigen- adjuvant composition. This is illustrated in Example 4. Follow-up compositions were used in which the Neogen is in the same form and dose as the priming immunization, but where the antigen is not present. As an alternative, the subject may be given several administrations of the antigen Neogen combination within a few days' time. Where the follow-up injections contain Neogen alone, the composition can be administered at or around the site of the priming immunization, so that the Neogen can further promote interaction between the immune system and the antigen previously administered.

Multiple administrations of Neogen may also have the benefit of promoting repopulation or activation of the immune system systemically, feeding into the reaction at the injection site that generates the specific response. In this context and for other reasons, the user may wish to test serum cytokine levels, cytokine production by circulating leukocytes, colony forming units in the spleen and in the bone marrow, reticulocytes in the blood, and other signs of hematopoiesis and immune activation.

Effective doses of vaccines are empirically determined, and may fall within the range of 10 to 500 µg of protein antigen, or 1 to 500 µg of nucleic acid, in combination with 10 to 1000 µg of adjuvant peptide, depending on size of the subject, immunogenicity of the antigen, and other factors. Suitable subjects include mammals of any kind, including research animals, livestock, pets, and human or non-human primates. Ultimate choice of the treatment protocol, dose, and monitoring is the responsibility of the managing clinician.

### EXAMPLES

### Example 1: Preparation of H-L-Ile-L-Glu-L-Trp-OH

The immunogenic peptides of the invention can generally be prepared using standard methods of peptide chemistry, such as those described in Chemistry of Peptide Synthesis by N. Leo Benoiton, CRC Press, 2005. The following illustration is adapted from Example 1 of PCT patent publication WO 2009/065217.

### Preparation of Boc-L-Glu(OBzl)-L-Trp-OMe

16.9 g (0.05 mol) of Boc-L-Glu(OBzl)-OH was dissolved in dioxane. 18.5 g (0.058 mol) of O-(1H-Benzotriazo-1-yl)-N,N,N',N'-tetramethyluronium tetrafluoroborate (TBTU) was then added to the solution and mixed well. 12.7 g (0.05 mol) of L-Trp-OMe•HCl and 25.3 mL (0.25 mol) of N-methylmorpholine (to pH ∼9-9.2) were also then added to the mixture. The suspension dissolved during the completion of the reaction after 12-18 hours at room temperature.

The solvents were evaporated in vacuo and the residual oil was dissolved in 250 mL of EtOAc, transferred into a separatory funnel and washed with 50 mL of 5% H₂SO₄, 2 x 50 mL of water, 150 mL of 5% NaHCO₃, and 3 x 50 mL of water to a neutral pH. The organic layer was separated and dried with anhydrous sodium sulfate. After drying, the EtOAc was evaporated in vacuo.

The residue was dissolved in the mixture of 150 mL of ethyl ether and 60 mL of hexane. A precipitate was formed, filtered off and washed with a mixture of 100 mL of ethyl ether and 50 mL of hexane and subsequently dried.

The yield was 21.5 g (79.9%) and had an R_{f} = 0.83 (CHCl₃:EtOAc:MeOH:AcOH = 6:3:1:0.1).

### Preparation of Fmoc-L-Ile-L-Glu(OBzL)-L-Trp-OMe

26.9 g (0.05 mol) of Boc-L-Glu(OBzl)-L-Trp-OMe was dissolved in 50 mL of dichloromethane. 50 mL of trifluoroacetic acid was added to the solution and the mixture was stirred for 40 min at room temperature. The solvent was evaporated in vacuo and the residual oil was dissolved in dioxane. N-methylmorpholine was then added to the mixture to a pH ∼9-9.2 (Solution 1)/

16.9 g (0.048 mol) of Fmoc-L-Ile-OH was dissolved in dioxane. 19.9 g (0.062 mol) of O-(1H-Benzotriazo-1-yl)-N,N,N',N'-tetramethyl-uronium tetrafluoroborate (TBTU) was added to the solution and mixed well. Solution 1 was then added to the mixture. The suspension dissolved during the completion of reaction after 12-18 hours at room temperature.

Solvents were evaporated in vacuo and the residual oil was dissolved in 250 mL of EtOAc, transferred into a separatory funnel and washed with 2 x 75 mL of 5% H₂SO₄, 3 x 50 mL of water, 150 mL of 5% NaHCO₃, and 3 x 50 mL of water to a neutral pH. The organic layer was separated and dried with anhydrous sodium sulfate. After drying, the EtOAc was evaporated in vacuum.

The residue was dissolved in 200 mL of hot EtOAc. A mixture of 300 mL of ethyl ether and 200 mL of hexane was then added to the solution. A precipitate was formed, filtered off, and washed with a mixture of 50 mL of ethyl ether and 50 mL of hexane and subsequently dried.

The yield was 28.5 g (73.8%) and had an R_{f} =0.85. (CHCl₃:EtOAc:MeOH=6:3:1)

### Preparation of H-L-Ile-L-Glu-L-Trp-ONa

100 mL of dichloromethane and 120 mL of isopropanol were added to 19.4 g (0.025 mol) of -L-Ile L-Glu(OBzl)-L-Trp-OMe. 24 mL of 3N NaOH was then added to the mixture. The suspension dissolved during the completion of the reaction after 3-4 hours at room temperature. The solvents were then evaporated in vacuo and the residual oil was dissolved in 200 mL of EtOAc and 200 mL of water, and transferred into a separatory funnel. The water layer was washed with 100 mL of EtOAc and separated and the pH of the solution was adjusted to 6.2 with acetic acid. The water solution was then evaporated in vacuo to a minimum volume. 600 mL of ethanol was then added to the residue. A precipitate was formed, filtered off, washed with ethanol and then dried.

The yield was 8.9 g (76.0%) and had an R_{f} =0.53 (CHCl₃:MeOH: 32% AcOH=5:3:1). Other peptides for use as an adjuvant according to this invention can be prepared in a similar fashion.

### Example 2: Immunomodulating properties of Neogen

The immunomodulating properties of Neogen were tested in intact animals and animals with secondary immunodeficiencies that were irradiation induced. This example is adapted from Examples 4 and 13 of U.S. Patent 6,159,940.

Female and male (CBA x C57BL) F1 mice, aged about 2.5 months weighing about 20 g, were irradiated with gamma-rays using a LUCh-1 apparatus. Immunological activity was assessed by antibody forming cell (AFC) count. T-cell count in spleen was determined by the method of spontaneous rosette formation with sheep erythrocytes (E-FRC).

Mice were irradiated in a dose of 2 Gy, the peptide was injected in the dose of 10 µg/kg according to the following scheme (to determine T-cell count by the method of spontaneous rosette formation): 1 time an hour after the irradiation; 2 times an hour, and a day after irradiation; 3 times an hour, a day, and two days after the irradiation; 4 times an hour, a day, two days and three days after the irradiation. The intact mice received the peptide 3 or 4 times, injected intramuscularly. The control group (2 Gy) received injections of physiological solution according to the same schedule. On completion of the treatment course, 10 mice from each group were immunized with sheep erythrocytes (SE) and 4-5 days later AFC counts were determined in their spleens. The rest of the mice were used to determine T-cell count by the method of spontaneous rosette formation. The state of the organs of the immune system (spleen and thymus) in mice with radiation immunodeficiency against the background of H-Ile-Glu-Trp-OH treatment was also evaluated by nucleated cell counts in thymus and spleen per mg of organ weight.

In the results obtained, the peptide injections to irradiated mice (one and four injections) brought about an increase in the karyocyte count in spleen per mg of organ weight and, a certain growth of the karyocyte count in thymus (3 and 4 injections). The number of antibody forming cells practically doubled in irradiated mice injected with the peptide (3 and 4 injections). T-cell count in spleen grew in all mice who received the peptide injections, especially three or four injections.

When inducing the humoral response to SE in intact mice, AFC increased 5 times, the T-cell count being the same as it was. We conclude that the peptide had a pronounced immunomodulating effect when injected both to intact and irradiated mice. There was a pronounced immunostimulating effect under radiation immunodeficiencies, and it is most effective when injected 3 to 4 times.

Action of H-Ile-Glu-Trp-OH was also studied in mixed lymphocyte culture (MLC) in an in vitro model of the reaction occurring in Graft Versus Host Disease. The reaction H-2d, anti H-2b was examined. Each variant was made in a triplet. Microcultures were incubated for 4 days, then ³H-thymidine was added; then the mixture was incubated for 16 more hours. It was then transferred to the filters, the amount of ³H-thymidine was determined. H-Ile-Glu-Trp-OH was added at the beginning of the incubation in different concentrations. At concentrations of 1, 10, and 20 µg/mL, the peptide stimulated proliferation of the allogeneic lymphocytes, while in concentrations of 0.1 µg/mL, there was negligible inhibition of the proliferation.

The effect of other model peptides was tested for their ability to protect hematopoietic cells against the effects of irradiation in an allograft. Donors were irradiated with 4 Gy from a ⁶⁰Co source, and used to prepare a suspension of bone marrow cells. The cells were irradiated at 4°C with 1 Gy of radioactivity at a rate of 0.8 Gy per minute, 5 to 10 minutes prior to injection into lethally irradiated recipients (8 Gy). Test peptides were injected intraperitoneally into the recipients at a dose of 10 ug per kg at 15-30 minutes after the irradiated bone marrow cells. Results are shown in Table 1.

| TABLE 1: Protection of Hematopoietic Activity Against the Effect of 1 Gy of Radiation by Test Peptides | |
|---|---|
| Peptide | Number of CFU-S |
| No irradiation | 10.2 ± 0.4 |
| Irradiation control | 5.6 ± 0.3 |
| Glu-Trp-OH | 10.5 ± 0.3 |
| iGlu-Trp-OH | 10.1 ± 0.5 |
| Pyro-Glu-Trp-OH | 9.3 ± 0.4 |
| Ile-Glu-Trp-OH | 11.5 ± 0.3 |
| Ile-Glu-(Trp)-OH | 8.5 ± 0.5 |
| Ile-Glu-Trp-NH₂ | 7.9 ± 0.4 |
| Leu-Glu-Trp-OH | 6.5 ± 0.5 |
| Val-Glu-Trp-OH | 8.2 ± 0.3 |
| Ala-Glu-Trp-OH | 8.7 ± 0.4 |
| Phe-Glu-Trp-OH | 6.6 ± 0.3 |
| Tyr-Glu-Trp-OH | 7.3 ± 0.4 |
| Lys-Glu-(Trp) -OH | 5.9 ± 0.5 |
| Lys-Glu-Trp-OH | 6.2 ± 0.4 |
| Lys-Glu-(Trp-NH₂)-OH | 5.9 ± 0.4 |

### Example 3: Influenza Vaccine Preparation

To determine the ability of Neogen to induce a specific immune response to a clinically important antigen, the following experiment was done with Vaxigrip® as immunogen, and Neogen® (H-Ile-Glu-Trp-OH) as adjuvant Test Article.

Vaxigrip® is an inactivated influenza vaccine trivalent Types A and B (split virion), manufactured and distributed by Sanofi Pasteur Limited, Toronto, Canada. It is prepared from virus grown in the allantoic cavity of embryonated eggs. The virus is purified by zonal centrifugation on a sucrose gradient, dissolved in the surfactant octoxinol 9 (Triton® X-100), inactivated in formaldehyde, and then diluted in phosphate buffered saline. It has traces of formaldehyde, octoxinol, and neomycin.

The strain used is adjusted when needed to stimulate a response against infectious strains prevailing in the general population. For the 2009-2010 season each 0.5 mL dose of Vaxigrip® contains 15 µg HA A/Brisbane/59/2007 (H1N1)-like strain [A/Brisbane/59/2007 (IVR-148)], 15 µg HA A/Brisbane/10/2007 (H3N2)-like strain [A/Uruguay/716/2007 (NYMC X-175C)], and 15 µg HA B/Brisbane/60/2008-like strain (B/Brisbane/60/2008). Other Ingredients are ≤30 µg formaldehyde, up to 0.5 mL sodium phosphate-buffered, isotonic sodium chloride solution, 2 µg thimerosal as preservative, the surfactant Triton® X-100, and trace amounts of sucrose and neomycin. There is no adjuvant present.

The formulations were prepared for this study with sterile, non-pyrogenic glassware, aids and materials under the laminar flow of HEPA filtered air according to the following procedures.

First, to prepare *Stock Solutions A, B, and* C (20 mg/mL, 0.5 mg/mL, and 0.02 mg/mL respectively), an appropriate amount of Neogen (the Test Article) was transferred to a volumetric flask (class A) of the appropriate volume. It was dissolved in sterile, non-pyrogenic 0.9% Sodium Chloride for injections, USP (the Vehicle), and made up to the proper volume. The solution was filtered through a sterile, non-pyrogenic PVDF membrane filter with porosity NMT 0.2 µm. The first 1/5 portion of the filtered solution was discarded. The filtered solution was dispensed into sterile, non-pyrogenic containers with an airtight closure system.

*Formulation I:* Vaxigrip alone. 222 µL of Vaxigrip suspension was transferred to a sterile, non-pyrogenic container with an airtight closure system, and diluted with the Vehicle to make 2000 µL and mixed by inversion. Each 100 µL of the Formulations I to VI contained about 1 µg of influenza virus hemagglutinin consisting of 0.33 µg of each of Influenza A H1N1, Influenza A H3N2, and Influenza B Florida/04/2006.

*Formulation II, III, and IV:* Vaxigrip plus 1 µg, 25 µg, or 100 µg of Neogen, respectively. 222 µL of Vaxigrip suspension was transferred to a sterile, non-pyrogenic container with an airtight closure system. 1000 of the Stock Solution C, B, or A respectively was added. The mixture was diluted with the Vehicle to make 2000 µL, and mixed by inversion.

*Formulation V.1* was made in the same manner as Formulation IV. *Formulation V.2* was made by transferring 2000 µL of Stock Solution A to a sterile, non-pyrogenic container with an airtight closure system, diluted with the Vehicle to make 4000 µL, and mixed by inversion. Each 100 µL of Formulation V.2 contained 100 µg of Neogen alone.

*Formulation VI:* Vaxigrip plus Alhydrogel® (aluminum hydroxide): 0.5 ml Alhydrogel (2% w/v) added to 2.0 ml Vehicle and vortexed briefly. It was pelleted by centrifugation (2000 X g for 10 minutes, and resuspended in 0.5 mL Vehicle. 1.11 mL Vaxigrip was then added, and suspended by rotation for 2 hours at room temperature. After refrigeration for 1 hour at 0-1 °C , the mixture was centrifuged at 2000 x g for 10 min. The pellet was resuspended in 10 mL vehicle for injection. Each 100 µL of Formulation VI contained approximately 10 µg of Alhydrogel.

*Formulation VII:* Neogen alone. 1000 µL of Stock Solution A were transferred to a sterile, non-pyrogenic container with an airtight closure system and diluted with the Vehicle to make 2000 µL. Each 100 µL of the Formulation (VII) contained 100 µg of the Test Article.

### Example 4: Use of Vaccine to Elicit a Specific Immune Response

Immunogenicity of the Formulations was determined at a test facility operated by the University Health Network, Toronto, Canada, under direction of Immunotech Designs Inc. (owner of this invention).

Female Balb/c mice 9-11 weeks of age (19-21 grams) were housed 5 mice per cage. Ten mice each were randomized into treatment groups. 100 µL of the appropriate Formulation for the allocated group was injected as a bolus subcutaneously on Day 1, and then again on Day 28. Group 5 received 100 µg of Formulation V.1 (Vaxigrip plus Neogen), and then 100 µL of Formulation V.2 (Neogen alone) 12 and 24 hours afterwards at the same site as Formulation V.1.

The mice were observed for activity level, posture, huddling, anorexia, dyspnea, neurological effects, lethargy, and reactions at the injection site. Body weighs were measured on Day 1, and weekly thereafter. Blood was collected on Days 14, 28, 35, and 42 from the saphenous vein without anticoagulants. Serum was separated and stored at -70 °C until assay

Blood was tested in a serial dilution hemagglutination inhibition (HI) assay. This measures the ability of antibodies induced in the immunized mice to inhibit agglutination of red cells caused by hemagglutinin on the influenza virus. HI titers were expressed as the reciprocal of the highest dilution of serum that inhibits hemagglutination. Specific antibody of the IgG₁ and IgG2a subclasses were determined by ELISA. Production of cytokines IL-2, IL-4, and IFNγ was determined from splenocytes taken from the spleens of four mice in each group following sacrifice on Day 42.

FIG. 1 shows the HI titers from the mice in each group, tested using influenza of the H1N2, H3N2, and B strains (mean ± standard deviation). Mice in Groups I to VI had substantial levels of antibody against each of the strains, showing that they were responding to all three viral components of the trivalent Vaxigrip.

There was no HI titer in Group VII receiving Neogen alone, showing that the peptide does not directly induce a immune response to the influenza. All of Groups I to IV and VI had a substantial and roughly similar HI titer. Apparently, the Vaxigrip on its own (Group I) ultimately produces a strong antibody response in mice, even in the absence of an added adjuvant. This is may be because Vaxigrip is a detergent extract of whole virus particles, in which the hemagglutinin and neuraminidase are inherently alloyed with viral components that promote both innate and adaptive immunity. Addition of the proven adjuvant Alhydrogel® (aluminum hydroxide) (Group VI) had no noticeable additional effect.

However, follow-up injections of Neogen alone following the Vaxigrip-Neogen combination boosts the anti-flu immune response above what is obtained by the various Vaxigrip preparations without the follow-up injections. As shown in FIG. 1, the mice in Group V, receiving Vaxigrip plus Neogen, and then 2 follow-up injections of Neogen, had a higher response than any of the other groups. The mean geometric titer in Group V was higher than Group I. The level in Group V ranked substantially higher than Groups I to IV and VI pooled together.

FIG. 2 shows the kinetics of H3N2 seroconversion in the various groups. Here, an individual animal was considered a seroconverter if HI titer showed a four-fold increase from baseline. Three of the Neogen adjuvant groups showed earlier seroconversion of a larger proportion of animals than either the flu antigen (Vaxigrip) alone, or the Alhydrogel composition. If this result holds true, it would constitute evidence that Neogen promotes a more rapid protective response.

FIG. 3 shows the IgG1 and IgG2a antibody response to influenza antigen, as determined by ELISA. Specific IgG1 is generally associated with a Th2 regulated response, whereas specific IgG2a is generally associated with a Th1 regulated response, which typically includes cellular immunity. As shown, the Alhydrogel preparation showed earlier stimulation of a Th2 response, which is consistent with the known tendency of aluminum salts to promote humoral (antibody) immunity in preference to a cellular response.

There was a statistically significant increase in IgG1 levels in the low-dose Neogen Vaxigen group compared with the group that received Vaxigen alone (area-under-the-curve analysis). Successively higher amounts of Neogen in the vaccine composition produced progressively lower IgG1 responses and higher IgG2a responses. With 100 µg of Neogen in the composition (Groups IV and V), the response on Day 35 and Day 42 was considerably more than the response to Vaxigen alone (Group I) or to Vaxigen plus Alhydrogel (Group VI).

These data suggest that polarization of the immune response to an immunogen can be modulated by the amount or dose level of Neogen, with lower doses favoring a Th2 response, and higher doses favoring a Th1 response. By using a high proportion of Neogen in the composition, the user may be able to generate a higher T cell response than is generally obtained using a standard vaccine.

### Example 5: Cell-based Assays Designed to Mimic the Peripheral Tissue Environment

Further experiments can be done using the MIMIC® immune response model designed and implemented by VaxDesign Corp. (Orlando FL). In the peripheral tissue equivalent (PTE) module, HUVEC endothelial cells are grown on a collagen matrix, and layered with peripheral blood mononuclear cells (PBMC) from different human donors. Monocytes migrate through the HUVEC cell layer, differentiating macrophages (which stay in the collagen matrix), and dendritic cells (which migrate back into the nutrient solution). In the subsequent lymphocyte tissue equivalent (LTE) module, the collected dendritic cells are co-cultured with lymphocytes in a manner designed to mirror interactions in a human lymph node in the presence of antigen.

The potency of Neogen as an adjuvant can be studied by comparing different Neogen concentrations in the PTE or LTE phase. Response to recombinant hemagglutinin in the presence of Neogen, alum, or no adjuvant can be compared with the response to a commercial influenza vaccine as a positive control. Output can be determined by measuring cell count and phenotype, production of cytokines in the LTE (such as GM-CSF, IL-1β, TNFα, IL-2, IL-4, IL-5, IL-6, IL-7, IL-8, IL-10, IL-12 (p70), IL-13, IFNα, and MCP-1), and the titer of specific antibody by ELISA and hemaglutination inhibition assay (HAI).

As shown in Figure 4, preliminary results of one such investigation were as follows: In the PTE, as little as 1 ug/mL of Neogen in the presence of antigen enhanced the ability of donor PBMCs to differentiate into macrophages and dendritic cells. Neogen in the presence of antigen increased the number of dendritic cells recovered from the system, whereas Neogen alone did not. Dendritic cells from PBMC cultured with both Neogen and antigen were primed for antigen presentation, as indicated by cell-surface expression of the antigen presenting protein HLA-DR. Culturing with Neogen plus antigen or Neogen alone resulted in fewer dendritic cells migrating across the HUVEC layer. To the extent this represents differentiation into macrophages rather than dendritic cells, this may indicate that Neogen also has an adjuvant effect on the innate immune system.

*The medicaments and their use as described in this disclosure can be modified effectively by routine experimentation and analysis without departing from the invention embodied in the claims that follow.*

## Claims

1. An immunogenic composition, comprising an antigen and an oligopeptide, wherein the oligopeptide is Ile-Glu-Trp and the antigen is an Influenza antigen. A

2. The composition of claim 1, wherein the oligopeptide has a peptide bond between the alpha carboxyl group on Glu and the amino group on Trp.

3. The composition of claim 1, wherein the oligopeptide had a peptide bond between the gamma carboxyl group on Glu and the ammo group on Trp.

4. The composition of any one of claims 1-3, wherein the antigen is in the form of a synthetic oligopeptide.

5. The composition of any one of claims 1-3, comprising a combination of antigens from a virus.

6. The composition of claim 5, wherein the antigen combination is presented on or within a live, attenuated, or inactivated viral or bacterial particle or extract thereof.

7. The composition of claim 5, wherein the antigen combination is a combination of antigens from different strains of a virus.

8. The composition of claim 5, wherein the antigen combination comprises one or more epitopes from neuraminidase and/or hemagglutinin of several strains of Influenza A, and optionally contains one or more epitopes from one or more strains of Influenza B and/or Influenza C.

9. The composition of any preceding claim, which produces a stronger Th1 or cellular immune response to the antigen than a composition comprising the same amount of antigen in an aluminum salt adjuvant.

10. A kit for eliciting an immune response according to claim 18 or 19, comprising an immunogenic composition comprising an antigen and an oligopeptide according to claims 1-9 in one container, and said oligopeptide without the antigen in another container.

11. A method for manufacturing the composition of claims 1-9, comprising combining said antigen with said peptide.

12. The immunogenic composition of any one of claims 1 to 7 for use in a method of eliciting a specific immune response against a particular antigen.

13. The immunogenic composition of any one of claims 1 to 7 for use in a method of treating a disease or infection in which said antigen is a component.

14. The immunogenic composition of any one of claims 1 to 7 for use in a method of generating a specific Th1 or cellular immune response against said antigen.

15. The immunogenic composition for use according to claim 12, wherein the cellular immune response is a Th1 or Th2 response.

## Patentansprüche

1. Immunogene Zusammensetzung, umfassend ein Antigen und ein Oligopeptid, wobei das Oligopeptid Ile-Glu-Trp ist und das Antigen ein Influenzaantigen ist.

2. Zusammensetzung nach Anspruch 1, wobei das Oligopeptid eine Peptidbindung zwischen der alpha-Carboxylgruppe auf Glu und der Aminogruppe auf Trp aufweist.

3. Zusammensetzung nach Anspruch 1, wobei das Oligopeptid eine Peptidbindung zwischen der gamma-Carboxylgruppe auf Glu und der Aminogruppe auf Trp aufweist.

4. Zusammensetzung nach einem der Ansprüche 1-3, wobei das Antigen in Form eines synthetischen Oligopeptids vorliegt.

5. Zusammensetzung nach einem der Ansprüche 1-3, umfassend eine Kombination von Antigenen aus einem Virus.

6. Zusammensetzung nach Anspruch 5, wobei die Antigenkombination auf oder in einem lebenden, attenuierten oder inaktivierten viralen oder bakteriellen Partikel oder Extrakt davon präsentiert wird.

7. Zusammensetzung nach Anspruch 5, wobei es sich bei der Antigenkombination um eine Kombination von Antigenen aus verschiedenen Stämmen eines Virus handelt.

8. Zusammensetzung nach Anspruch 5, wobei die Antigenkombination ein oder mehrere Epitope aus Neuraminidase und/oder Hämagglutinin von mehreren Stämme von Influenza A umfasst und wahlweise ein oder mehrere Epitope aus einem oder mehreren Stämmen von Influenza B und/oder Influenza C enthält.

9. Zusammensetzung nach einem der vorhergehenden Ansprüche, die eine stärkere Th1- oder zelluläre Immunreaktion auf das Antigen erzeugt als eine Zusammensetzung, die die gleiche Menge an Antigen in einem Aluminumsalzadjuvans umfasst.

10. Kit zum Herbeiführen einer Immunreaktion nach Anspruch 18 oder 19, umfassend eine immunogene Zusammensetzung, umfassend ein Antigen und ein Oligopeptid nach den Ansprüchen 1-9 in einem Behälter und das Oligopeptid ohne das Antigen in einem anderen Behälter.

11. Verfahren zur Herstellung der Zusammensetzung aus den Ansprüchen 1-9, umfassend das Kombinieren des Antigens mit dem Peptid.

12. Immunogene Zusammensetzung nach einem der Ansprüche 1 bis 7 zur Verwendung in einem Verfahren zum Herbeiführen einer spezifischen Immunreaktion gegen ein bestimmtes Antigen.

13. Immunogene Zusammensetzung nach einem der Ansprüche 1 bis 7 zur Verwendung in einem Verfahren zum Behandeln einer Infektionskrankheit, wobei das Antigen ein Bestandteil ist.

14. Immunogene Zusammensetzung nach einem der Ansprüche 1 bis 7 zur Verwendung in einem Verfahren zum Erzeugen einer spezifischen Th1- oder zellulären Immunreaktion gegen das Antigen.

15. Immunogene Zusammensetzung zur Verwendung nach Anspruch 12, wobei es sich bei der zellulären Immunreaktion um eine Th1- oder Th2-Reaktion handelt.

## Revendications

1. Composition immunogène, comprenant un antigène et un oligopeptide, dans laquelle l'oligopeptide est Ile-Glu-Trp et l'antigène est un antigène de la grippe.

2. Composition selon la revendication 1, dans laquelle l'oligopeptide possède une liaison peptidique entre le groupe alpha-carboxyle sur Glu et le groupe amino sur Trp.

3. Composition selon la revendication 1, dans laquelle l'oligopeptide possède une liaison peptidique entre le groupe gamma-carboxyle sur Glu et le groupe amino sur Trp.

4. Composition selon l'une quelconque des revendications 1 à 3, dans laquelle l'antigène est sous la forme d'un oligopeptide synthétique.

5. Composition selon l'une quelconque des revendications 1 à 3, comprenant une combinaison d'antigènes provenant d'un virus.

6. Composition selon la revendication 5, dans laquelle la combinaison d'antigènes est présentée sur ou au sein d'une particule virale ou bactérienne vivante, atténuée ou inactivée ou un extrait de celle-ci.

7. Composition selon la revendication 5, dans laquelle la combinaison d'antigènes est une combinaison d'antigènes provenant de différentes souches d'un virus.

8. Composition selon la revendication 5, dans laquelle la combinaison d'antigènes comprend un ou plusieurs épitopes provenant de neuraminidase et/ou d'hémagglutinine de plusieurs souches de grippe A, et contient éventuellement un ou plusieurs épitopes provenant d'une ou plusieurs souches de grippe B et/ou de grippe C.

9. Composition selon l'une quelconque des revendications précédentes, qui produit une réponse immunitaire de Th1 ou cellulaire à l'antigène plus forte qu'une composition comprenant la même quantité d'antigène dans un adjuvant de type sel d'aluminium.

10. Trousse pour provoquer une réponse immunitaire selon la revendication 8 ou 9, comprenant une composition immunogène comprenant un antigène et un oligopeptide selon les revendications 1 à 9 dans un contenant, et ledit oligopeptide sans l'antigène dans un autre contenant.

11. Procédé de fabrication de la composition selon les revendications 1 à 9, comprenant la combinaison dudit antigène avec ledit peptide.

12. Composition immunogène selon l'une quelconque des revendications 1 à 7, utilisable dans un procédé pour provoquer une réponse immunitaire spécifique vis-à-vis d'un antigène particulier.

13. Composition immunogène selon l'une quelconque des revendications 1 à 7, utilisable dans un procédé de traitement d'une maladie ou d'une infection dans laquelle ledit antigène est un composant.

14. Composition immunogène selon l'une quelconque des revendications 1 à 7, utilisable dans un procédé de production d'une réponse immunitaire spécifique de Th1 ou cellulaire vis-à-vis dudit antigène.

15. Composition immunogène utilisable selon la revendication 12, dans laquelle la réponse immunitaire cellulaire est une réponse de Th1 ou de Th2.
